(19) Europäisches Patentamt
European Patent Office
Office européen des brevets

(11) EP 0 968 159 B1

(12) # EUROPÄISCHE PATENTSCHRIFT

(45) Veröffentlichungstag und Bekanntmachung des Hinweises auf die Patenterteilung:
**10.10.2001 Patentblatt 2001/41**

(21) Anmeldenummer: **98904089.4**

(22) Anmeldetag: **17.01.1998**

(51) Int Cl.[7]: **C07C 1/22**, C07C 15/46, C07C 35/02

(86) Internationale Anmeldenummer:
**PCT/EP98/00244**

(87) Internationale Veröffentlichungsnummer:
**WO 98/32719 (30.07.1998 Gazette 1998/30)**

(54) **VERFAHREN ZUR HERSTELLUNG VON STYROLEN**
METHOD FOR PREPARATION OF STYRENES
PROCEDE POUR PREPARER DES STYRENES

(84) Benannte Vertragsstaaten:
**AT BE DE ES FR GB IT NL**

(30) Priorität: **29.01.1997 DE 19703111**

(43) Veröffentlichungstag der Anmeldung:
**05.01.2000 Patentblatt 2000/01**

(73) Patentinhaber: **Aventis Research & Technologies GmbH & Co KG**
**65926 Frankfurt am Main (DE)**

(72) Erfinder:
- **ROESKY, Rainer**
**D-65929 Frankfurt am Main (DE)**
- **BORCHERT, Holger**
**D-67278 Bockenheim (DE)**
- **DINGERDISSEN, Uwe**
**D-64342 Seeheim-Jugenheim (DE)**

(74) Vertreter: **Ackermann, Joachim, Dr.**
**Postfach 11 13 26**
**60048 Frankfurt am Main (DE)**

(56) Entgegenhaltungen:
**DE-A- 2 317 525** **US-A- 3 636 182**

- **HOLLEMAN WIBERG: "Lehrbuch der Anorganischen Chemie" 1985 , DE GRUYTER , BERLIN XP002064376 in der Anmeldung erwähnt siehe Seite 881-882**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 968 159 B1

Printed by Jouve, 75001 PARIS (FR)

## Beschreibung

Die vorliegende Erfindung betrifft ein technisch einfaches Verfahren zur Herstellung von substituierten Styrolen in Gegenwart von Oxidkatalysatoren, insbesondere Metalloxidkatalysatoren.

Bedingt durch die große Bedeutung der substituierten Styrole in technischer und präparativer Chemie z.B. Verwendung als Monomere sowie als Copolymere, in der Polystyrolherstellung (z.B. p-Hydroxystyrol Herstellung (ACS Symp.Ser.412(1989)), gibt es zahlreiche Methoden zu ihrer Herstellung. Die Verfahren zur gezielten Herstellung reiner, isomerenfreier, substituierter Styrole sind jedoch, soweit vorhanden, häufig nicht optimal.

So ist z.B. bekannt, daß man aus Methylhalogeniden und substituierten Toluolen oberhalb von 700°C in der Gasphase Styrole herstellen kann (US-PS 3 636 182). Die bei dem beschriebenen Verfahren notwendigen hohen Temperaturen, bis zu 1200 °C, begünstigen Nebenreaktionen sowie Zersetzungsreaktionen. Ferner kommt es während der Reaktion zur Bildung von Salzsäure, so daß der Reaktor aus Materialien bestehen muß, die auch unter diesen extremen Bedingungen nicht angegriffen werden. Nachteilig ist insbesondere der unvollständige Umsatz und die geringe Selektivität des hier offenbarten Verfahrens.

Ferner kann man substituierte Styrole auch aus Ethylbenzolen durch katalytische Dehydrierung herstellen (DE-OS 2 317 525). Nachteilig bei diesem Verfahren ist der meist unvollständige Umsatz des Ausgangsmaterials und die damit verbundene Abtrennung des nichtumgesetzten Ausgangsmaterials vom Produkt durch Destillation. Dehydrierungen der hier beschriebenen Art sind nur in Spezialapparaturen durchführbar und darüber hinaus sind Ethylbenzole in technischen Mengen nur schwer verfügbar.

Die Herstellung von p-substituierten Styrolderivaten durch 1. Umsetzung von parasubstituierten Benzylhalogeniden mit Triphenylphosphin in Chloroform und 2. Anschliessender Reaktion mit Formalinlösung und Natriumhydroxid wird in Synth.Commun. 6(1976)53 beschrieben. Da die Umsetzung in Lösung erfolgt, kommt es auch bei diesem Verfahren nach Beendigung der Reaktion zu Abtrennungsproblemen bei der Isolierung des Endproduktes.

Die im Stand der Technik beschriebenen Verfahren zeigen keine zufriedenstellenden Ergebnisse hinsichtlich des Umsatzes der Ausgangsstoffe oder der Selektivität der Reaktion. Ferner sind die beschrieben Verfahren oft mit hohen Temperaturen und einem relativ großen Aufwand zur Gewinnung des Reinproduktes verbunden.

Es bestand somit die Aufgabe, ein einfaches und wirtschaftliches Verfahren zur selektiven Herstellung von Styrolen zu finden, das die aus dem Stand der Technik bekannten Nachteile vermeidet.

Es wurde nun gefunden, daß Acetophenone mit Wasserstoff an einem Oxidkatalysator in einem Reaktionsschritt zu Styrolen überführt werden können.

Die vorliegende Erfindung betrifft somit ein Verfahren zur selektiven Herstellung eines Styrols der Formel (1)

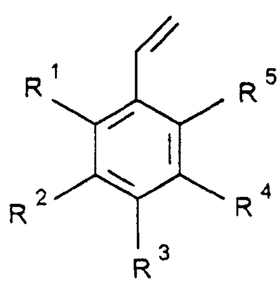

(1)

durch Umsetzung eines Acetophenons der Formel (2) mit Wasserstoff

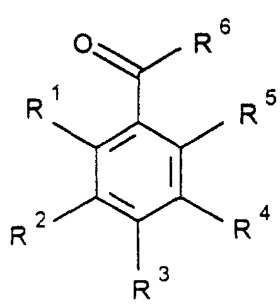

(2)

in Gegenwart eines Katalysators enthaltend eines oder mehrere Oxide, insbesondere Metalloxide, worin das Oxid ein Element der Gruppen Ia, IIa, IIIa, IVa, Va, Via, Ib, IIb, IIIb, Ivb, Vb, VIb, VIIb und/oder VIII oder ein Element der Gruppe der Lanthaniden enthält.

Die Reste $R^1$, $R^2$, $R^3$, $R^4$, $R^5$ und $R^6$ in den Formeln (1) und (2) sind gleich oder verschieden und stehen für H, F, Cl, Br oder I oder für einen $C_1$-$C_{30}$-kohlenstoffhaltigen, gegebenenfalls Heteroatome enthaltenden Rest, insbesondere für eine ($C_1$-$C_{20}$)-Alkyl-, ($C_6$-$C_{14}$)-Aryl, ($C_1$-$C_{10}$)-Alkoxy-,($C_2$-$C_{10}$)-Alkeny-1, ($C_7$-$C_{20}$)-Arylalkyl-, ($C_7$-$C_{20}$)-Alkylaryl-, ($C_6$-$C_{10}$)-Aryloxy-, ($C_1$-$C_{10}$)-Fluoralky-l, ($C_6$-$C_{10}$)-Halogenaryl- oder eine ($C_2$-$C_{10}$)-Alkinylgruppe oder einen Rest -$SiR^7_3$, wobei $R^7$ für ($C_1$-$C_{10}$)-Alkyl steht, oder zwei oder mehr Reste $R^1$ bis $R^5$ bilden mit den sie verbindenden Atomen einen oder mehrere substutierte oder unsubstituierte Ringe oder $R^4$ und $R^5$ stehen zusammen für einen aromatischen Ring.

Beispiele für heteroaromatische Reste sind Thienyl, Furyl oder Pyridyl.

Die von benachbarten Resten $R^1$ bis $R^7$ gebildeten Ringe können durch Substituenten in der Bedeutung von $R^1$ bis $R^6$, einschließlich der dafür genannten Vorzugsbereiche, substituiert sein.

In der Formel (1) gilt bevorzugt, daß R1, R2, R3, R4 und R5 gleich oder verschieden sind und Wasserstoff, (C6-C10)-Aryl oder (C1-C10)-Alkyl bedeuten, oder die Reste R1 und R2, R2 und R3, R3 und R4 oder R4 und R5 mit den sie verbindenden Atomen einen substituierten oder unsubstituierten sechsgliedrigen, gesättigten oder ungesättigten Carbocyclus bilden. $R^3$ steht vorzugsweise für Isopropyl und $R^6$ für Methyl.

Der von benachbarten Substituenten $R^1$-$R^4$ gebildete gesättigte oder ungesättigte Fünf- oder Sechsring (Carbocyclus) kann zusätzlich Substituenten, bevorzugt ($C_1$-$C_{10}$)-Alkyl, tragen.

Beispiele für Styrole der Formel (2) sind Styrol, 2-Isobutylstyrol, 3-Isobutylstyrol, 3-Isobutyl-1-methylstyrol, 4-Isobutylstyrol, 4-Isobutylmethylstyrol, 4-Isobutylphenylstyrol, 2-Isopropylstyrol, 2-Isopropyl-5-methylstyrol, 4-Isopropylstyrol, 4-Isobutylphenylstyrol, 4-Isobutyl-3-methylstyrol, 5-Isopropyl-3-methylstyrol, 5-Isopropyl-2-methylstyrol, Vinylnaphthalin oder 4-Methylvinylnaphthalin.

Als Katalysator werden bevorzugt solche eingesetzt, die eines oder mehrere Oxide der allgemeinen Formel (3) enthalten,

$$Zn_aM^1{}_bM^2{}_cO_x \qquad (3)$$

worin

$M^1$ für mindestens ein Element aus der Gruppe Al, Zr, Ti und Si steht;
$M^2$ für mindestens ein Element aus der Gruppe Cd, Cu, Ag, Ni, Co, Fe, Mn, Cr, Mo, Ta, Sc, W, V, Nb, Hf, Yt, B, In, Zn, Pb, Bi, Se, Ga, Ge, Sb, As, Te oder ein Element der Lanthanide steht. Die Indices a, b, c und x stehen für die Grammatomverhältnisse und liegen vorzugsweise in folgenden Bereichen:

a = 1,
b =0 - 20, insbesondere 0,01 - 15
c = 0 - 3, insbesondere 0,01 bis 2,5 und

x ist die zum Stöchiometrieausgleich notwendige Anzahl von Sauerstoffatomen, die sich entsprechend der Oxidationszustände von Zn, $M^1$ und $M^2$ ergibt;

In einer weiteren bevorzugten Ausführungsvariante der vorliegenden Erfindung kann der Katalysator anstatt der Oxide der Formel (3) Oxide der Formel (4) enthalten,

$$M^3{}_2M^4O_4 \qquad (4)$$

bei denen es sich um Doppeloxide aus der Verbindungsklasse der Spinelle handelt (Chemie der Elemente, N.N. Greenwood, A. Earnshaw, VCH-Verlagsgesellschaft mbH, Weinheim, 1990; Lehrbuch der Anorganischen Chemie, A.F. Holleman, N.Wiberg, de Gruyter, Berlin, 1985). In Formel (4) können $M^3$ und $M^4$ jedes beliebige Element des Periodensystems der Elemente darstellen, wobei $M^3$ ein Element mit der Oxidationsstufe I, II oder III darstellt und $M^4$ ein Element mit der Oxidationsstufe II, III, oder VI ist. Bevorzugterweise tritt folgendes auf: wenn $M^3$ ein Element mit der Oxidationsstufe I darstellt, besitzt $M^4$ die Oxidationsstufe VI; wenn $M^3$ ein Element mit der Oxidationsstufe II darstellt, besitzt $M^4$ die Oxidationsstufe IV und wenn $M^3$ ein Element mit der Oxidationsstufe III darstellt, besitzt $M^4$ die Oxidationsstufe II. Es ist ebenfalls möglich, daß es sich bei $M^3$ und $M^4$ um das gleiche Element handelt, sofern es in den entsprechenden, unterschiedlichen Oxidationsstufen vorliegen kann.
Insbesondere enthält der Katalysator folgende Oxide der Formel (4): $Al_2ZnO_4$, $Al_2CoO_4$, $Al_2MnO_4$, $Al_2FeO_4$, $Al_2NiO_4$ und $Cr_2FeO_4$.

Die erfindungsgemäß eingesetzten Katalysatoren sind in der prioritätsälteren nicht vorveröffentlichten Deutschen Anmeldung P 196 088 14.3 (HOE 96/F 054) für die Herstellung von Indenen aus Indanon beschrieben worden.

Der erfindungsgemäß verwendete Katalysator kann ein anorganisches oder organisches Trägermaterial enthalten. Als Trägermaterialien werden vorzugsweise folgende Materialien verwendet: Aluminiumoxide, Siliciumdioxid, Alumosilkate, Titandioxid, Zirkoniumdioxid, Thoriumdioxid, Lanthanoxid, Magnesiumoxid, Calciumoxid, Bariumoxid, Zinnoxid, Cerdioxid, Zinkoxid, Boroxid, Bornitrid, Borcarbid, Borphosphat, Zirkoniumphosphat, Siliziumnitrid oder Siliziumcarbid oder Polypyridine oder Polyacrylate.

Der Katalysator kann durch Imprägnierung auf einen Träger oder Formkörper oder durch Cofällung sowie anschließender Trocknung und Kalzination hergestellt werden. Eine weitere Möglichkeit besteht in der direkten Kalzination geeigneter Metallverbindungen, z.B. von Nitraten, Acetaten, Carbonaten oder anderen Salzen und Komplexen der Elemente $M^1$ bis $M^4$.

Zur Imprägnierung auf Träger kann eine Lösung von Verbindungen der Elemente $M^1$ bis $M^4$ auf einen Träger, der bevorzugt anorganisch ist und beispielsweise aus $SiO_2$, SiC, $Al_2O_3$, $Al(OH)_3$, $ZrO_2$, Alumosilikaten, SiN oder $TiO_2$ bestehen kann, aufgebracht werden. Zur Imprägnierung geeignete Verbindungen der Elemente $M^1$ bis $M^4$ sind z.B. deren Halogenide, Nitrate, Sulfate, Oxalate, Carboxylate und Alkoxide. Die imprägnierten Träger werden anschließend bei 100°C bis 170°C, bevorzugt bei 120°C bis 150°C, getrocknet und bei 400-1000°C, bevorzugt bei 500 bis 800°C kalziniert. Die so hergestellten Katalysatoren können nach üblichen Verfahren vor oder nach dem Kalzinieren zu Pellets, Tabletten oder Extrudaten weiterverarbeitet werden.

Zur Cofällung können geeignete Verbindungen der Elemente $M^1$ bis $M^4$ bei geeigneten pH-Werten gefällt werden. Nach der Fällung werden entstandenen Hydroxide abfiltriert und mit einem geeigneten Lösungsmittel gewaschen. Die Trocknung erfolgt bei 100 bis 170°C, bevorzugt bei 120°C bis 150 °C, gegebenenfalls unter Anlegung von Vakuum, die Kalzination bei 400 bis 1000°C, bevorzugt bei 500 bis 900°C. Der so hergestellte Katalysator liegt als Granulat vor und kann nach Zerkleinerung auf die gewünschte Teilchengröße direkt in der Reaktion eingesetzt werden.

Vor dem Einsetzen in dem erfindungsgemäßen Verfahren, kann der Katalysator bei Temperaturen von 100 bis 800°C mit einem geeignetem Reduktionsmittel präformiert werden.

Das erfindungsgemäße Verfahren kann in kontinuierlicher oder diskontinuierlicher Fahrweise in einem geeigneten Reaktor durchgeführt werden. Vorzugsweise werden bei dem erfindungsgemäßen Verfahren Verbindungen der Formel (2) bei einer Temperatur im Bereich von 200 bis 600°C, insbesondere bei 250 bis 400°C, und einem Druck im Bereich von 0,1 bis 10 bar, insbesondere bei atmosphärischem Druck, in Gegenwart eines Katalysators enthaltend Verbindungen der Formel (3) oder Verbindungen der Formel (4) zu Verbindungen der Formel (1) umgesetzt. Die Reaktionszeiten liegen üblicherweise im Bereich von 0,5 bis 10 Stunden, insbesondere im Bereich von 1 bis 6 Stunden.

Das vorliegende Verfahren kann mit molekularem Wasserstoff durchgeführt werden, der auch in situ hergestellt werden kann. Es ist auch möglich den Wasserstoff mit einem Inertgas wie Stickstoff oder Argon zu verdünnen. Das erfindungsgemäß eingesetzte Acetophenon kann als Feststoff, als Schmelze, als Flüssigkeit oder als Lösung in einem geeigneten Lösungsmittel, wie Benzol, Xylol, Toluol oder Cyclohexan eingesetzt werden. Bevorzugt wird das Isopropylacetophenon einem Verdampfer zugeführt und anschließend in der Gasphase mit dem erfindungsgemäß eingesetzten Katalysator in Kontakt gebracht. Das molare Verhältnis des Acetophenons zu Wasserstoff liegt bevorzugt bei 1:1 bis 1:500, besonders bevorzugt bei 1:2 bis 1:50. Die Zufuhrgeschwindigkeit für die Acetophenone liegt bevorzugt bei 0,01 bis 20 g/ml Katalysator (LHSV: liquid hourly space velocity), besonders bevorzugt bei 0,2 bis 10 g/ml Katalysator. Die Zufuhrgeschwindigkeit des Wasserstoffs liegt bevorzugt bei 50 bis 50000 $h^{-1}$ (GHSV: gaseous hourly space velocity), besonders bevorzugt bei 100 bis 10000 $h^{-1}$.

Die mit dem beschriebenen Verfahren hergestellten Styrole können von Nebenprodukten durch Destillation, Säulenchromatographie oder Kristallisation gereinigt werden.

Im Unterschied zu den bekannten Verfahren zur Herstellung von Styrol ermöglicht das erfindungsgemäße Verfahren den Erhalt von reinen, isomerenfreien Styrolen aus leicht verfügbaren Ausgangsstoffen in einem Schritt. Da das Verfahren unter relativ milden Reaktionsbedingungen durchgeführt wird, wird die Bildung von unerwünschten Neben- oder Zerfallsprodukten minimiert. Die erfindungsgemäß erhaltenen Styrole können ohne großen Aufwand vom Reaktionsgemisch abgetrennt und anschließend gereinigt werden.

Mit Hilfe des beschriebenen Verfahrens können Styrole der Formel (1) mit einer Selektivität im Bereich von 80 bis 100%, insbesondere von 85 bis 99 % hergestellt werden. Die Umsätze für Acetophenon liegen hierbei üblicherweise im Bereich von 80 bis 100 %, insbesondere im Bereich von 85 bis 97%.

Die Erfindung wird anhand der nachfolgenden Beispiele erläutert. Der Umfang der aufgeführten Beispielen hat jedoch keinerlei limitierenden Charakter:

Beispiele:

Beispiel 1:

139,0g $Al(NO_3)_3$ x 9 $H_2O$ und 55,0 g $Zn(NO_3)_2$ x 6 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 2:

69,5g $Al(NO_3)_3$ x 9 $H_2O$ und 110,0 g $Zn(NO_3)_2$ x 6 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 3:

139,0g $Al(NO_3)_3$ x 9 $H_2O$ und 110,0 g $Zn(NO_3)_2$ x 6 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 4:

139,0g $Al(NO_3)_3$ x 9 $H_2O$ und 54,9 g $Zn(NO_3)_2$ x 6 $H_2O$ und 2,2 g $Co(NO_3)_3$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 5:

139,0g $Al(NO_3)_3$ x 9 $H_2O$ und 54,9 g $Zn(NO_3)_2$ x 6 $H_2O$ und 1,35 g $Cu(NO_3)_2$ x 3 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 6:

139,0g $Al(NO_3)_3$ x 9 $H_2O$ und 54,9 g $Zn(NO_3)_2$ x 6 $H_2O$ und 2,23 g $Cr(NO_3)_3$ x 9 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 7:

139,0g $Al(NO_3)_3$ x 9 $H_2O$ und 54,9 g $Zn(NO_3)_2$ x 6 $H_2O$ und 1,62 g $Ni(NO_3)_3$ x 6 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 8:

139,0g $Al(NO_3)_3$ x 9 $H_2O$ und 54,9 g $Zn(NO_3)_2$ x 6 $H_2O$ und 1,40 g $Mn(NO_3)_3$ x 4 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 9:

139,0g $Al(NO_3)_3$ x 9 $H_2O$ und 54,9 g $Zn(NO_3)_2$ x 6 $H_2O$ und 2,03 g $Y(NO_3)_3$ x 6 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 10:

139,0g $Al(NO_3)_3$ x 9 $H_2O$ und 54,9 g $Zn(NO_3)_2$ x 6 $H_2O$ und 2,25 g $Fe(NO_3)_3$ x 9 $H_2O$ werden in 3,8 l Wasser aufgelöst und auf 5°C abgekühlt. Der pH-Wert mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 11

139,0g $Al(NO_3)_3$ x 9 $H_2O$ und 54,9 g $Zn(NO_3)_2$ x 6 $H_2O$ und 2,42 g $Ce(NO_3)_3$ x 6 $H_2O$ werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiel 12:

139,0g $Al(NO_3)_3$ x 9 $H_2O$ und 54,9 g $Zn(NO_3)_2$ x 6 $H_2O$ und 1,16 g Tetraethoxysilan werden in 3,8 l Wasser aufgelöst und auf 5°C abgekühlt. Der pH-Wert wird mit 25%iger Ammoniaklösung auf pH =9 eingestellt. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

Beispiele 13-18:

In einem Rohrreaktor wurde 4-Isopropylacetophenon in Gegenwart der Katalysatoren aus den Beispielen 1-4 mit Wasserstoff zu 2-Isopropylstyrol umgesetzt. Das Schüttvolumen des eingesetzten Katalysators betrug 20 ml. Die Verdampfung des eingesetzten 4-Isopropylacetophenons erfolgte in einem vorgeschalteten Verdampfer. GHSW (gaseous hourly space velocity), LHSV (liquid hourly space velocity) und Reaktionstemperatur sind in Tabelle 1 angegeben. Das Reaktionsprodukt wurde mit einem Kühler kondensiert und gaschromatographisch analysiert.

Tabelle 1:

| Bsp. | Katalysator aus Bsp. | GHSV | LHSV | Temperatur | Umsatz | Selektivität |
|---|---|---|---|---|---|---|
| 13 | 1 | 25 | 5 | 300 | 100 | 88 |
| 14 | 2 | 25 | 4,5 | 330 | 100 | 86 |
| 15 | 3 | 25 | 5,5 | 330 | 100 | 87 |
| 16 | 4 | 25 | 9,5 | 330 | 100 | 85 |
| 17 | 1 | 25 | 10 | 350 | 100 | 87 |
| 18 | 2 | 25 | 7,5 | 300 | 100 | 78 |
| 19 | 3 | 25 | 2,6 | 350 | 100 | 91 |
| 20 | 4 | 25 | 2,5 | 300 | 100 | 74 |

Beispiel 19-20:

In einem Rohrreaktor wurde 4-Isobutylacetophenon in Gegenwart der Katalysatoren aus den Beispielen 3 und 4 mit Wasserstoff zu 2-Isobutylstyrol umgesetzt. Das Schüttvolumen des eingesetzten Katalysators betrug 20 ml. Die Verdampfung des eingesetzten 4-Isobutylacetophenons erfolgte in einem vorgeschalteten Verdampfer. GHSW, LHSV und Reaktionstemperatur sind in Tabelle 1 angegeben. Das Reaktionsprodukt wurde mit einem Kühler kondensiert und gaschromatographisch analysiert.

Beispiele 21-22:

In einem Rohrreaktor wurden 4-Isobutylacetophenon in Gegenwart von ZnO mit Wasserstoff zu 2-Isobutylstyrol umgesetzt. Das Schüttvolumen des eingesetzten Katalysators betrug 20ml. Die Verdampfung des eingesetzten 4-Isobutylacetophenons erfolgte in einem vorgeschalteten Verdampfer. GHSW, LHSV und Reaktionstemperatur sind in Tabelle 2 angegeben. Das Reaktionsprodukt wurde mit einem Kühler kondensiert und gaschromatographisch analysiert.

Tabelle 2:

| Bsp. | Katalysator | GHSV | LHSV | Temperatur | Umsatz | Selektivität |
|---|---|---|---|---|---|---|
| 21 | ZnO | 25 | 2,7 | 350 | 100 | 95 |
| 22 | ZnO | 25 | 2,3 | 300 | 100 | 99 |

**Patentansprüche**

1. Verfahren zur selektiven Herstellung eines Styrols der Formel (1)

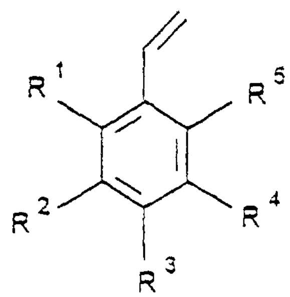

(1)

durch Umsetzung eines Acetophenons der Formel (2) mit Wasserstoff

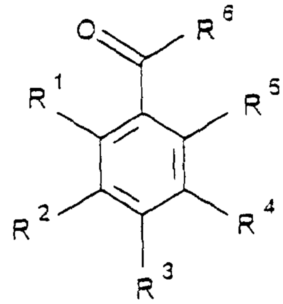

(2)

worin $R^1$, $R^2$, $R^3$, $R^4$, $R^5$, und $R^6$ gleich oder verschieden sind und für H, F, Cl, Br oder I oder für einen $C_1$-$C_{30}$-kohlenstoffhaltigen, gegebenenfalls Heteroatome enthaltenden Rest stehen, insbesondere für eine ($C_1$-$C_{20}$)-Alkyl-, ($C_6$-$C_{14}$)-Aryl, ($C_1$-$C_{10}$)-Alkoxy-,($C_2$-$C_{10}$)-Alkeny-l, ($C_7$-$C_{20}$)-Arylalkyl-, ($C_7$-$C_{20}$)-Alkylaryl-, ($C_6$-$C_{10}$)-Aryloxy-,

$(C_1-C_{10})$-Fluoralky-l, $(C_6-C_{10})$-Halogenaryl- oder eine $(C_2-C_{10})$-Alkinylgruppe oder einen Rest $-SiR^7_3$, wobei $R^7$ für $(C_1-C_{10})$-Alkyl steht, oder wobei zwei oder mehr Reste $R^1$ bis $R^5$ können mit den sie verbindenden Atomen einen oder mehrere substituierte oder unsubstituierte Ringe bilden,
in Gegenwart eines Katalysators enthaltend eines oder mehrere Oxide, insbesondere Metalloxide, worin das Oxid ein Element der Gruppen Ia, IIa, IIIa, IVa, Va, VIa, Ib, IIb, IIIb, IVb, Vb, VIb, VIIb und/oder VIII oder ein Element der Gruppe der Lanthaniden enthält.

2. Verfahren nach Anspruch 1, **dadurch gekennzeichnet, daß** der Katalysator eines oder mehrere Oxide der allgemeinen Formel (3) enthält

$$Zn_aM^1{}_bM^2{}_cO_x \qquad (3)$$

worin

$M^1$ für mindestens ein Element aus der Gruppe Al, Zr, Ti und Si steht
$M^2$ für mindestens ein Element aus der Gruppe Cd. Cu. Ag, Ni, Co, Fe, Mn, Cr, Mo, Ta, Sc, W, V, Nb, Hf, Yt, B, In, Zn, Pb, Bi, Se, Ga. Ge, Sb, As. Te oder ein Element der Lanthanide steht,
a = 1 ,  b =0 - 20,  c = 0 - 3
x ist die zum Stöchiometrieausgleich notwendige Anzahl von Sauerstoffatomen, die sich entsprechend der Oxidationszustände von Zn, $M^1$ und $M^2$ ergibt.

3. Verfahren nach Anspruch 1 , **dadurch gekennzeichnet, daß** der Katalysator Verbindungen der Formel (4) enthält

$$M^3{}_2M^4O_4 \qquad (4)$$

worin

$M^3$ ein Element mit der Oxidationsstufe I, II oder III ist und
$M^4$ ein Element mit der Oxidationsstufe II, III, oder VI ist.

4. Verfahren nach Anspruch 3, **dadurch gekennzeichnet, daß** $M^3$ ein Element mit der Oxidationsstufe I und $M^4$ ein Element mit der Oxidationsstufe VI ist.

5. Verfahren nach mindestens einem der Ansprüche 1 bis 4, **dadurch gekennzeichnet, daß** der Katalysator Verbindungen der Formel $Al_2ZnO_4$, $Al_2CoO_4$, $Al_2MnO_4$, $Al_2FeO_4$, $Al_2NiO_4$ und/oder $Cr_2FeO_4$ enthält.

6. Verfahren nach mindestens einem der Ansprüche 1 bis 5, **dadurch gekennzeichnet, daß** der Katalysator ein anorganisches oder organisches Trägermaterial enthält.

7. Verfahren nach Anspruch 6, **dadurch gekennzeichnet, daß** das anorganische oder organische Trägermaterial aus der folgenden Gruppe ausgewählt ist: Aluminiumoxide, Siliciumdioxid, Alumosilkate, Titandioxid, Zirkoniumdioxid, Thoriumdioxid, Lanthanoxid, Magnesiumoxid, Calciumoxid, Bariumoxid, Zinnoxid, Cerdioxid, Zinkoxid, Boroxid, Bornitrid. Borcarbid, Borphosphat, Zirkoniumphosphat, Siliziumnitrid oder Siliziumcarbid oder Polypyridine oder Polyacrylate.

8. Verfahren nach mindestens einem der Ansprüche 1 bis 7, **dadurch gekennzeichnet, daß** Selektivität bei der Herstellung von Verbindungen der Formel (1) im Bereich von 80 bis 100% liegt.

9. Verfahren nach mindestens einem der Ansprüche 1 bis 8, **dadurch gekennzeichnet, daß** Verbindungen der Formel (2) bei einer Temperatur im Bereich von 200 bis 600 °C und einem Druck im Bereich von 0,1 bis 10 bar in Gegenwart eines Katalysators enthaltend Verbindungen der Formel (3) oder Verbindungen der Formel (4) mit Wasserstoff umgesetzt werden.

10. Verfahren nach mindestens einem der Ansprüche 1 bis 9, **dadurch gekennzeichnet, daß** das molare Verhältnis von Verbindungen der Formel (2) zu Wasserstoff im Bereich von 1:1 bis 1:500 liegt.

**11.** Verfahren nach mindestens einem der Ansprüche 1 bis 10, **dadurch gekennzeichnet, daß** die Zufuhrgeschwindigkeit des Wasserstoffs im Bereich von 50 bis 50000 $h^{-1}$ (GHSV: gaseous hourly space velocity) liegt.

**12.** Verfahren nach mindestens einem der Ansprüche 1 bis 11, **dadurch gekennzeichnet, daß** die Verbindung der Formel (1) Styrol, 2-Isobutylstyrol, 3-Isobutylstyrol, 3-Isobutyl-1-methylstyrol, 4-Isobutylstyrol, 4-Isobutylmethylstyrol, 4-Isobutylphenylstyrol, 2-Isopropylstyrol, 2-Isopropyl-5-methylstyrol, 4-Isopropylstyrol, 4-Isobutylphenylstyrol, 4-Isobutyl-3-methylstyrol, 5-lsopropyi-3-methylstyrol, 5-Isopropyl-2-methylstyrol, Vinyinaphthalin oder 4-Methylvinylnaphthalin ist.

**13.** Verfahren nach mindestens einem der Ansprüche 1 bis 12, **dadurch gekennzeichnet, daß** der Umsatz von Verbindungen der Formel (2) im Bereich von 80 bis 100 % liegt.

## Claims

**1.** A process for the selective preparation of a styrene of the formula (I)

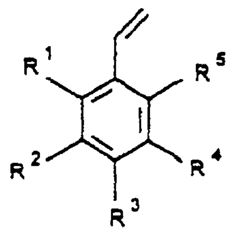

(1)

by reaction of an acetophenone of the formula (2)

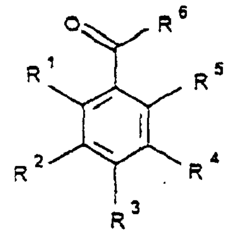

(2)

where $R^1$, $R^2$, $R^3$, $R^4$, $R^5$ and $R^6$ are identical or different and are each H, F, Cl, Br or I or a $C_1$-$C_{30}$-group which may contain heteroatoms, in particular a ($C_1$-$C_{20}$)-alkyl, ($C_6$-$C_{14}$)-aryl, ($C_1$-$C_{10}$)-alkoxy, ($C_2$-$C_{10}$)-alkenyl, ($C_7$-$C_{20}$)-arylalkyl, ($C_7$-$C_{20}$)-alkylaryl, ($C_6$-$C_{10}$)-aryloxy, ($C_1$-$C_{10}$)-fluoroalkyl, ($C_6$-$C_{10}$)-haloaryl or ($C_2$-$C_{10}$)-alkynyl group or an $-SiR^7_3$ radical, where $R^7$ is ($C_1$-$C_{10}$)-alkyl, or two or more of the radicals $R^1$ to $R^5$ together with the atoms connecting them may form one or more substituted or unsubstituted rings, with hydrogen in the presence of a catalyst comprising one or more oxides, in particular metal oxides, where the oxide comprises an element of groups Ia, IIa, IIIa, IVa, Va, Via, Ib, IIb, IIIb, IVb, Vb, VIb, VIIb and/or VIII or an element of the lanthanide series.

**2.** The process as claimed in claim 1, wherein the catalyst comprises one or more oxides of the formula (3),

$$Zn_aM^1{}_bM^2{}_cO_x \qquad (3)$$

where

$M^1$ is at least one element selected from the group consisting of Al, Zr, Ti and Si;
$M^2$ is at least one element selected from the group consisting of Cd, Cu, Ag, Ni, Co, Fe, Mn, Cr, Mo, Ta, Sc, W, V, Nb, Hf, Yt, B, In, Zn, Pb, Bi, Se, Ga, Ge, Sb, As, Te and the lanthanide elements,

a = 1, b = 0-20, c = 0-3,
x is the number of oxygen atoms necessary to balance the charge, which is determined by the oxidation states of Zn, $M^1$ and $M^2$.

**3.** The process as claimed in claim 1, wherein the catalyst comprises compounds of the formula (4)

$$M^3{}_2M^4O_4 \quad (4)$$

where

$M^3$ is an element in the oxidation state I, II or III and
$M^4$ is an element in the oxidation state II, III or VI.

**4.** The process as claimed in claim 3, wherein $M^3$ is an element in the oxidation state I and $M^4$ is an element in the oxidation state VI.

**5.** The process as claimed in at least one of claims 1 to 4, wherein the catalyst comprises compounds of the formulae $Al_2ZnO_4$, $Al_2CoO_4$, $Al_2MnO_4$, $Al_2FeO_4$, $Al_2NiO_4$, and/or $Cr_2FeO_4$.

**6.** The process as claimed in at least one of claims 1 to 5, wherein the catalyst comprises an inorganic or organic support material.

**7.** The process as claimed in claim 6, wherein the inorganic or organic support material is selected from the following groups: aluminum oxides, silicon dioxide, aluminosilicates, titanium dioxide, zirconium dioxide, thorium dioxide, lanthanum oxide, magnesium oxide, calcium oxide, barium oxide, tin oxide, cerium dioxide, zinc oxide, boron oxide, boron nitride, boron carbide, boron phosphate, zirconium phosphate, silicon nitride or silicon carbide or polypyridines or polyacrylates.

**8.** The process as claimed in at least one of claims 1 to 7, wherein the selectivity in the preparation of compounds of the formula (1) is in the range from 80 to 100%.

**9.** The process as claimed in at least one of claims 1 to 8, wherein compounds of the formula (2) are reacted with hydrogen at a temperature in the range from 200 to 600°C and a pressure in the range from 0.1 to 10 bar in the presence of a catalyst comprising compounds of the formula (3) or compounds of the formula (4).

**10.** The process as claimed in at least one of claims 1 to 9, wherein the molar ratio of compounds of the formula (2) to hydrogen is in the range from 1:1 to 1:500.

**11.** The process as claimed in at least one of claims 1 to 10, wherein the feed rate of the hydrogen is in the range from 50 to 50,000 $h^{-1}$ (GHSV: gas hourly space velocity).

**12.** The process as claimed in at least one of claims 1 to 11, wherein the compound of the formula (1) is styrene, 2-isobutylstyrene, 3-isobutylstyrene, 3-isobutyl-1-methylstyrene, 4-isobutylstyrene, 4-isobutylmethylstyrene, 4-isobutylphenylstyrene, 2-isopropylstyrene, 2-isopropyl-5-methylstyrene, 4-isopropylstyrene, 4-isobutylphenylstyrene, 4-isobutyl-3-methylstyrene, 5-isopropyl-3-methylstyrene, 5-isopropyl-2-methylstyrene, vinylnaphthalene or 4-methylvinylnaphthalene.

**13.** The process as claimed in at least one of claims 1 to 12, wherein the conversion of compounds of the formula (2) is in the range from 80 to 100%.

**Revendications**

**1.** Procédé pour la préparation sélective d'un styrène de formule (1)

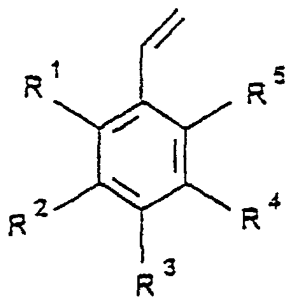

(1)

par réaction d'une acétophénone de formule (2) avec l'hydrogène

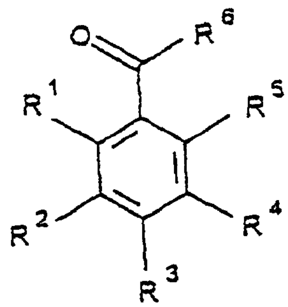

(2)

où $R^1$, $R^2$, $R^3$, $R^4$, $R^5$ et $R^6$ sont identiques ou différentes et représentent des atomes d'hydrogène, de fluor, de chlore, de brome ou d'iode ou un reste carboné en $C_1$-$C_{30}$, éventuellement contenant des hétéroatomes, en particulier un groupe alkyle en $C_1$-$C_{20}$, aryle en $C_6$-$C_{14}$, alkoxy en $C_1$-$C_{10}$, alcényle en $C_2$-$C_{10}$, arylalkyle en $C_7$-$C_{20}$, alkylaryle en $C_7$-$C_{20}$, aryloxy en $C_6$-$C_{10}$, fluoralkyle en $C_1$-$C_{10}$, haloaryle en $C_6$-$C_{10}$ ou un groupe alcynyle en $C_2$-$C_{10}$, ou un reste de formule $-SiR^7_3$, où $R^7$ représente un groupe alkyle en $C_1$-$C_{10}$, ou deux ou plus de deux restes $R^1$ à $R^5$ pouvant former avec les atomes les reliant un ou plusieurs cycles substitués ou non substitués,
en présence d'un catalyseur contenant un ou plusieurs oxydes, en particulier des oxydes métallifères, où l'oxyde contient un élément des groupes Ia, IIa, IIIa, IVa, Va, VIa, Ib, IIb, IIIb, IVb, Vb, VIb, VIIb et/ou VIII ou un élément du groupe des lanthanides.

2. Procédé selon la revendication 1, **caractérisé en ce que** le catalyseur contient un ou plusieurs oxydes de formule générale (3)

$$Zn_aM^1_bM^1_cO_x \quad (3)$$

où

$M^1$ représente au moins un élément du groupe Al, Zr, Ti et Si,
$M^2$ représente au moins un élément pris dans le groupe comportant Cd, Cu, Ag, Ni, Co, Fe, Mn, Cr, Mo, Ta, Sc, W, V, Nb, Hf, Yt, B, In, Zn, Pb, Bi, Se, Ga, Ge, Sb, As, Te ou un élément des lanthanides a = 1, b = 0-20, c = 0-3
x représente le nombre d'atomes d'oxygène nécessaire à une compensation stoechiométrique résultant conformément aux états d'oxydation de Zn, $M^1$ et $M^2$.

3. Procédé selon la revendication 1 **caractérisé en ce que** le catalyseur contient des composés de formule (4)

$$M^3_2M^4O_4 \quad (4)$$

où

$M^3$ représente un élément ayant le degré d'oxydation I, II ou III et

$M^4$ représente un élément ayant le degré d'oxydation II, III ou VI.

**4.** Procédé selon la revendication 3, **caractérisé en ce que** $M^3$ représente un élément ayant le degré d'oxydation I et $M^4$ un élément ayant le degré d'oxydation VI.

**5.** Procédé selon au moins l'une des revendications 1 à 4, **caractérisé en ce que** le catalyseur contient des composés de formule $Al_2ZnO_4$, $Al_2CoO_4$, $Al_2MnO_4$, $Al_2FeO_4$, $Al_2NiO_4$ et/ou $Cr_2FeO_4$.

**6.** Procédé selon au moins l'une des revendications 1 à 5, **caractérisé en ce que** le catalyseur contient une matière-support inorganique ou organique.

**7.** Procédé selon la revendication 6, **caractérisé en ce que** la matière-support inorganique ou organique est prise dans le groupe comportant : des oxydes d'aluminium, le dioxyde de silicium, des aluminosilicates, le dioxyde de titane, le dioxyde de zirconium, le dioxyde de thorium, l'oxyde de lanthane, l'oxyde de magnésium, l'oxyde de calcium, l'oxyde de baryum, l'oxyde d'étain, le dioxyde de cérium, l'oxyde de zinc, l'oxyde de bore, le nitrure de bore, le carbure de bore, le phosphate de bore, le phosphate de zirconium, le nitrure de silicium ou le carbure de silicium ou des polypyridines ou polyacrylates.

**8.** Procédé selon au moins l'une des revendications 1 à 7, **caractérisé en ce que** la sélectivité dans la préparation de composés de formule (1) se trouve dans le domaine de 80 à 100 %.

**9.** Procédé selon au moins l'une des revendications 1 à 8, **caractérisé en ce qu'**on fait réagir des composés de formule (2) à une température dans le domaine de 200 à 600°C et une pression dans le domaine de 0,1 à 10 bars, en présence d'un catalyseur contenant des composés de formule (3) ou composés de formule (4) avec l'hydrogène.

**10.** Procédé selon au moins l'une des revendications 1 à 9, **caractérisé en ce que** le rapport molaire des composés de formule (2) à l'hydrogène se trouve dans le domaine de 1:1 à 1:500.

**11.** Procédé selon au moins l'une des revendications 1 à 10, **caractérisé en ce que** la vitesse d'alimentation en hydrogène se trouve dans le domaine de 50 à 50 000 $h^{-1}$ (GHSV : gaseous hourly space velocity).

**12.** Procédé selon au moins l'une des revendications 1 à 11, **caractérisé en ce que** le composé de formule (1) est le styrène, le 2-isobutylstyrène, le 3-isobutylstyrène, le 3-isobutyl-1-méthylstyrène, le 4-isobutylstyrène, le 4-isobutylméthylstyrène, le 4-isobutylphénylstyrène, le 2-isopropylstyrène, le 2-isopropyl-5-méthylstyrène, le 4-isopropylstyrène, le 4-isobutylphénylstyrène, le 4-isobutyl-3-méthylstyrène, le 5-isopropyl-3-méthylstyrène, le 5-isopropyl-2-méthylstyrène, le vinylnaphtalène ou le 4-méthylvinylnaphtalène.

**13.** Procédé selon au moins l'une des revendications 1 à 12, **caractérisé en ce que** la conversion des composés de formule (2) se trouve dans le domaine de 80 à 100 %.